# EUROPEAN PATENT SPECIFICATION

(11) **EP 0 590 140 B1**
(45) Date of publication and mention of the grant of the patent: **25.11.1998**
(21) Application number: 93910621.7
(22) Date of filing: 14.04.1993
(51) Int. Cl.: A61M 29/02

(54) **LOW PROFILE DILATATION CATHETER**
DILATATIONSKATHETER MIT GERINGEM QUERSCHNITT
CATHETER DE DILATATION EXTRA-PLAT

(30) Priority: 20.04.1992 US 870820
(43) Date of publication of application: 06.04.1994
(60) Divisional application: 98200582.9
(73) Proprietor: ADVANCED CARDIOVASCULAR SYSTEMS, INC., Santa Clara California 95052-8167 (US)
(72) Inventor: SIRHAN, Motasim, M., Sunnyvale, CA 94087 (US); THORNTON, Troy, L., Foster City, CA 94404 (US); CAMPBELL, Patrick K., Cupertino,California 95014 (US)
(74) Representative: Pendlebury, Anthony
(86) International application number: US9303580
(87) International publication number: WO9320882

(56) References cited:
- EP-A- 0 374 859
- EP-A- 0 441 384
- US-A- 3 915 171
- US-A- 4 877 031
- US-A- 4 892 519

## Description

This invention generally relates to intravascular catheters, such as balloon dilatation catheters used in percutaneous transluminal coronary angioplasty (PTCA).

In classic PTCA procedures, a guiding catheter having a preshaped distal tip is percutaneously introduced into the cardiovascular system of a patient and advanced therein until the preshaped distal tip of the guiding catheter is disposed within the aorta adjacent the ostium of the desired coronary artery. The guiding catheter is twisted or torqued from the proximal end to turn the distal tip of the guiding catheter so that it can be guided into the coronary ostium. A guidewire and a balloon dilatation catheter are introduced into and advanced through the guiding catheter to the distal tip thereof, with the guidewire slidably disposed within an inner lumen of the dilatation catheter. The guidewire is first advanced out the distal tip of the guiding catheter, which is seated in the ostium of the patient's coronary artery, until the distal end of the guidewire crosses the legion to be dilated. The dilatation catheter is then advanced out of the distal tip of the guiding catheter, over the previously advanced guidewire, until the balloon on the distal extremity of the dilatation catheter is properly positioned across the lesion. Once properly positioned, the balloon is inflated to a predetermined size with radiopaque liquid at relatively high pressures (e.g., generally 405-1216 kN/m² [4-12 atmospheres]) to dilate the stenosed region of the diseased artery. The balloon is then deflated so that the dilatation catheter can be removed from the dilated stenosis and blood flow will resume therethrough.

Further details of guiding catheters, dilatation catheters, guidewires, and other devices for angioplasty procedures can be found in U.S. Patent 4,323,071 (Simpson-Robert); U.S. Patent 4,439,185 (Lundquist); U.S. Patent 4,468,224 (Enzmann *et al*.); U.S. Patent 4,516,972 (Samson); U.S. Patent 4,438,622 (Samson *et al*.); U.S. Patent 4,554,929 (Samson *et al*.); U.S. Patent 4,582,185 (Samson); U.S. Patent 4,616,652 (Simpson); U.S. Patent 4,638,805 (Powell); U.S. Patent 4,748,986 (Morrison *et al*.); U.S. Patent 4,898,577 (Badger *et al*.); and U.S.Patent 4,827,943 (Taylor *et al*.).

The assignee of the present invention, Advanced Cardiovascular Systems, Inc., markets an improved dilatation catheter under the trademarks ACS RX® which is described and claimed in U.S. Patent 5,040,548 (Yock), U.S. Patent 5,061,273 (Yock), and U.S. Patent 4,748,982 (Horzewski *et al*.). This dilatation catheter has a short guidewire receiving sleeve or inner lumen extending through a distal portion of the catheter. The sleeve or inner lumen extends proximally a distance of at least about 10 cm and usually not more than about 50 cm from a first guidewire port in the distal end of the catheter to a second guidewire port in the catheter spaced proximally from the inflatable member of the catheter. Preferably, a slit is provided in the wall of the catheter body which extends distally from the second guidewire port, preferably to a location proximal to the proximal end of the inflatable balloon. The structure of the catheter allows for the rapid exchange of the catheter without the need for an exchange wire or adding a guidewire extension to the proximal end of the guidewire. The design of this catheter has widely praised by the medical profession and has been met with much success in the market place because of the advantages of its unique design.

Another modification, which was introduced into the market place be the assignee of the present application provides a plurality of perfusion ports in the wall forming at least part of the catheter body proximal to the balloon. These perfusion ports are in fluid communication with an inner lumen extending to the distal end of the catheter body. A plurality of perfusion ports are preferably provided in the catheter body distal to the balloon which are also in fluid communication with the inner lumen extending to the distal end of the catheter body. When the balloon on the distal extremity of the dilatation catheter is inflated to dilate a stenosis, oxygenated blood in the artery or the aorta or both, depending upon the location of the dilatation catheter within the coronary anatomy, is forced to pass through the proximal perfusion ports, through the inner lumen of the catheter body and out the distal perfusion ports. This provides oxygenated blood downstream from the inflated balloon to thereby prevent or minimize ischemic conditions in tissue distal to the catheter. As is appreciated by those skilled in the art, tissue distal to a stenosis is frequently already in jeopardy due to ischemic conditions which may exist. As a result, care must be exercised in sizing the perfusion ports and the inner lumen to ensure that there is adequate flow of oxygenated blood to tissue distal to the catheter to eliminate or minimize ischemic conditions.

US-A-4892519 discloses a catheter as described above. The catheter comprises inner and outer tubular members which define two lumens for the passage of blood and inflating fluid, respectively. At least a portion of the inner periphery of the outer tubular member is secured to the exterior of the inner tubular member.

A major and continual thrust of development work in the field of intravascular catheters, particularly angioplasty catheters, has been to reduce the profile, *i.e.* transverse dimensions, of such catheters and to improve the flexibility thereof without detrimentally affecting the pushability, particularly in the distal portion, of such catheters. A reduction in profile with no loss in pushability allows an intravascular catheter to be advanced much further into a patient's vasculature and to cross much tighter lesions in the case of angiolplasty catheters.

Despite many advances in this field, the need for lower profile intravascular catheters having greater flexibility with little or no loss in pushability remains. The present invention satisfies this need.

The present invention relates to an elongated catheter for performing an angioplasty procedure comprising an elongated catheter shaft which has a distal shaft section with a first inner tubular member having a distal end, a port in the distal end and a guidewire-receiving inner lumen in fluid communication with the port in the distal end, and an outer tubular member being disposed about the first inner tubular member and having a portion of its inner periphery, along a length of the distal shaft section, secured to the exterior of the first inner tubular member and a dilation balloon on the distal shaft section distal to at least a portion of said length and having an interior chamber therein, characterized in that the catheter further comprises a second inner tubular member within the distal shaft section which has a distal end and a first port in the distal end, which is parallel to and off-set from the first inner tubular member, which extends to a location in the distal shaft section spaced proximally from the distal end of the first inner tubular member, which has an inflation lumen extending therein in fluid communication with the interior chamber of the dilation balloon through the port in the distal end of the second inner tubular member and which has a portion of the inner periphery of the outer tubular member along said length secured to an exterior portion of the second inner tubular member, the catheter shaft is provided with a second port which is located closer to the dilation balloon than the proximal end of the catheter shaft and which is in communication with the inner lumen of the first inner tubular member, the distal shaft section has along the length thereof one transverse dimension in a first direction substantially larger than a second transverse dimension in a second direction perpendicular to the first direction, and from 5% to 90% of the inner periphery of the outer tubular member is secured to the exterior of the first inner tubular member.

The intravascular catheter of the invention generally includes, at least in the distal portion thereof, an inner tubular member having an inner lumen extending therein and an outer tubular member disposed about the inner tubular member. A substantial part of the distal portion of the outer tubular member is secured or bonded to the inner tubular member along a length thereof. Preferably, the secured part is shaped to conform to the shape of the outer surface of the inner tubular member. Along this length at least about 5% to about 90%, preferably about 30% to about 80%, of the peripheral of the outer tubular member secured to the inner tubular member. The portion of the outer tubular member which is not secured to the inner tubular member along said length forms at least in part a longitudinally extending inner lumen. The bond between the inner tubular member and the conforming portion of the outer tubular member need not be continuous along the length and may be intermittent so long as a significant portion of the outer tubular member is secured to the inner tubular member. The length of the secured section should not be less than 5 mm and is preferably about 10 to about 40 cm. While in a presently preferred embodiment described herein the secured section of the outer tubular member is limited to the distal section of the catheter, the secured section may extend along essentially the entire length of the catheter.

The distal portion of the catheter may be provided with a further diagnostic or treatment means, other than the inflatable dilatation balloon for angioplasty, distal to the secured section.

By securing a substantial part of the outer tubular member to the exterior of the inner member along a length thereof to form a fused coaxial design in at least the distal portion of the catheter, the profile of the catheter body in at least one transverse dimension in that area is reduced substantially to thereby provide improved flexibility in at least one direction. Moreover, the secured portions of the inner member and the outer tubular members support one another to provide improvements in the pushability of the catheter. By virtue of the transverse dimension in one direction being substantially larger than the transverse dimension in a second direction at right angles to the first direction the cross-sectional shape of the catheter provides substantial improvements in flexibility, trackability and pushability. The maximum dimension of the catheter shaft should be at least 15% greater than the minimum dimension, preferably at least 25% greater.

Maximum cross-sectional dimensions of the small diameter section of the outer tubular member for coronary dilatation catheters are on the order of about 0.02 to about 0.06 in (0.51 - 1.5 mm). For peripheral arteries this dimension may be larger.

With catheter designs of the invention having the capability for rapid exchangeability, as described in the previously described Horzewski *et al.* patent, there is a tendency for the peel-away slit to open up or expand upon the introduction of the inflation fluid at high pressures into the inflation lumen defined by the unsecured portion of the outer tubular member. To avoid this problem the inflation lumen defined by the unsecured part of the distal section is provided with a support tube so that there is essentially no expansion of the inflation lumen and therefore there is little tendency for the peel-away slit to open up or expand.

The improvements of the invention are applicable to a wide range of intravascular catheters and particularly to essentially all types of dilatation catheters with inflatable or expandable members, such as those described in the patents identified herein. These and other advantages of the invention will become more apparent from the following detailed description of the invention when taken in conjunction with accompanying exemplary drawings.

The invention will now be described, by way of example only, with reference to the accompanying drawings in which:-
Fig. 1 is an elevational view, partially in section, of a balloon dilatation catheter not in accordance with the invention.
Fig. 2 is a transverse cross-sectional view of the catheter shown in Fig. 1 taken along the lines 2-2.
Fig. 3 is a transverse cross-sectional view of the catheter shown in Fig. 1 taken along the lines 3-3.
Fig. 4 is a transverse cross-sectional view of the catheter shown in Fig. 1 taken along the lines 4-4.
Fig. 5 is a transverse cross-sectional view of the catheter shown in Fig. 1 taken along the lines 5-5.
Fig. 6 is an elevational view, partially in section, of another dilatation catheter not in accordance with the invention.
Fig. 7 is a transverse cross-sectional view of the catheter shown in Fig. 6 taken along the lines 7-7.
Fig. 8 is a transverse cross-sectional view of the catheter shown in Fig. 6 taken along the lines 8-8.
Fig. 9 is a transverse cross-sectional view of the catheter shown in Fig. 6 taken along the lines 9-9.
Fig. 10 is a transverse cross-sectional view of the catheter shown in Fig. 6 taken along the lines 10-10.
Fig. 11 is an elevational view, partially in section, of another dilatation catheter not in accordance with the invention.
Fig. 12 is a transverse cross-sectional view of the catheter shown in Fig. 11 taken along the lines 12-12.
Fig. 13 is a transverse cross-sectional view of the catheter shown in Fig. 11 taken along the lines 13-13.
Fig. 14 is a transverse cross-sectional view of the catheter shown in Fig. 11 taken along the lines 14-14.
Fig. 15 is a transverse cross-sectional view of the catheter shown in Fig. 11 taken along the lines 15-15.
Fig. 16 is an elevational view, partially in section, of a catheter in accordance with the present invention. The catheter is a modification of the catheter shown in Figs. 6-10.
Fig. 17 is a transverse cross-sectional view of the embodiment shown in Fig. 16, taken along the lines 17-17.
Fig. 18 is a transverse cross-sectional view of a modification of the catheter shown in Fig. 1 wherein two inner lumens are provided in the distal section between the inner and outer tubular members.

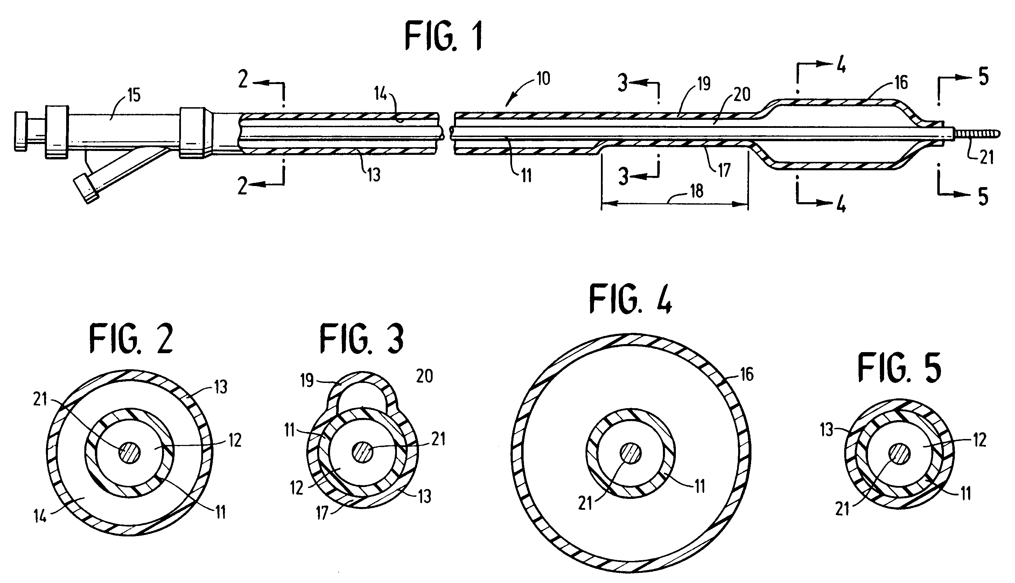

Figs. 1-5 schematically illustrate an over-the-wire dilatation catheter not in accordance with the invention. The catheter includes an elongated catheter shaft 10 which has an inner tubular member 11 with an inner lumen 12, an outer tubular member 13 disposed about the inner tubular member and defining therebetween an annular inner lumen 14 which extends through the proximal portion of the catheter shaft. An adapter 15 is secured to the proximal end of the elongated catheter body 10. A relatively inelastic, inflatable balloon 16 is formed as part of the outer tubular member 13 with the distal end of the balloon secured to the distal end of the inner tubular member 11. The balloon 16 may be formed from the same tubing as the outer tubular member 13 as shown in Fig. 1 or it may be made separately and secured to the distal end of a tubular shaft which forms part of the outer tubular member.

The outer tubular member 13 generally has a distal section 17 which is secured to the exterior of the inner tubular member 11 as best shown in Figs. 1 and 3 along a length 18 to provide smaller transverse dimensions in at least one direction. The distal section 17 is secured to the exterior of the inner tubular member 11 with a significant portion of the periphery outer member along the length 18, typically about 50% to about 80%, being secured to the inner member. The unsecured portion 19 of the distal section 17 along the length 18 forms an inflation lumen 20 which is in fluid communication with the interior of the balloon 16 and the annular lumen 14.

The use of the dilatation catheter shown in Figs. 1-5 may generally follow conventional PTCA practices with over-the-wire dilatation catheters. In the conventional practices a guidewire 21 is backloaded into the inner lumen 12 of the inner tubular member 11 of the catheter body 10 and both the guidewire and the catheter are advanced together through a guiding catheter (not shown) which has been previously disposed within the patient's arterial system, with the distal end of the guiding catheter seated within the ostium of the desired coronary artery. The guidewire 21 is first advanced out the distal end of the guiding catheter into the patient's coronary anatomy until it extends beyond the lesion to be dilated, and then the dilatation catheter is advanced over the guidewire which is being held in its position, until the balloon 16 on the dilatation catheter is properly disposed within the stenotic region so that the lesion is dilated upon the inflation of the balloon. After dilatation, the balloon 16 is deflated and the catheter and the guidewire 21 are withdrawn from the patient. If further treatment or diagnosis is to be conducted, the guidewire 21 can be replaced with an exchange wire before removing the dilatation catheter so that the first catheter can he removed and another advanced into the desired location or an extension wire can be attached to the proximal end of the guidewire in place to perform essentially the sane function. For further details see the discussion of exchange wires and extension wires in U.S. Patent 4,827,941 (Taylor *et al*.).

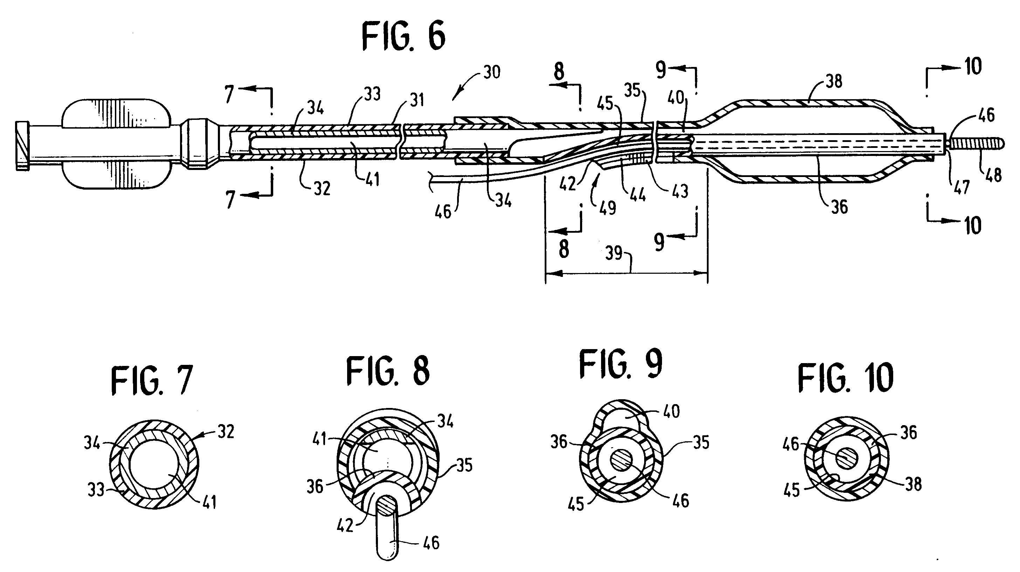

Figs. 6-10 schematically illustrate another dilatation catheter not in accordance with the invention which is quite similar in its most distal structure to the embodiment shown in Figs. 1-5. In the embodiment shown in Figs. 6-10, the catheter shaft 30 includes an outer tubular member 31 which has a two layered proximal portion 32 with an outer plastic tubular jacket or coating 33 which fits tightly, *e.g.* is shrunk fit, into a tubular element 34 which may be formed of hypotubing. The outer tubular member 31 also includes a distal section 35 which is secured about the inner tubular member 36 as in the previous embodiment. The distal section 35 may be the proximal skirt of the balloon 38, as shown in the drawing, or it may be a separate tubular member. The relatively inelastic balloon 38 is secured by its distal end to the distal end of the inner tubular member 36 which extends through the interior thereof. A significant portion of the interior surface of the distal section 35 along the length 39 is secured to the exterior of the inner tubular member 36 in accordance with the requirements of the invention. The inner tubular member 36 of this embodiment is quite short compared to the inner tubular of the embodiment shown in Figs. 1.5. The unsecured portion of the distal section 35 forms an inflation lumen 40 which is in fluid communication with the lumen 41 in the proximal section 32 of the outer tubular member 31 and the interior of the balloon 38.

In the embodiment shown in Figs. 6-10, the catheter body 30 is provided a guidewire port 42 which passes through the secured walls 43 and 44 of the inner tubular member 36 and the distal section 35 of the outer tubular member 31 respectively and which is in communication with a relatively short inner lumen 45 extending within the inner tubular member 36. Guidewire 46 extends through the inner lumen 45 and out the proximal port 42 and a distal guidewire port 47. A coil 48 is provided on the distal end of the guidewire 46.

The inner tubular element 34 onto which the outer plastic tubular element 33 is secured is preferably hypotubing and may be formed of conventional stainless steel or a NiTi alloy, particularly a NiTi alloy with superelastic properties.

The above-described catheter construction with a relatively short inner lumen 45, adapted to slidably receive the guidewire 46, eliminates the need for using an exchange wire or a guidewire extension. A peel-away slit 49 is preferably provided in the secured walls 43 and 44 of the inner and outer tubular members 36 and 31 which extend from the guidewire port 42 to a location proximal to the proximal end of the balloon 38. The peel-away slit 49 facilitates the removal of the catheter from the proximal end of the guidewire 46 when the catheter is to be replaced or exchanged for another catheter and it also eliminates the need for using a guidewire extension or an exchange wire as described in Horzewski *et al*.

A dual lumen type construction such as described in Horzewski *et al*. may also be used in the portion of the catheter proximal to the guidewire port 42.

There are at least two modes of inserting the dilatation catheter shown in Figs. 6-10 into the patient's coronary anatomy. The first method is for the most part the same as in the previously described catheter, namely, the guidewire 46 is preloaded into the short inner lumen 45 of the inner tubular member 36 of the catheter body 30 and both are advanced through a guiding catheter (not shown) previously disposed within the patient's arterial system with the distal end of the guiding catheter seated within the ostium of a coronary artery. The second mode, frequently called the "bare wire" technique, involves first advancing a guidewire 46 through and out the guiding catheter until it is positioned within the patient's coronary artery across the lesion to be dilated. The proximal end of the guidewire 46, which is outside the patient, is backloaded, *i.e.* inserted into the short inner lumen 45 of the inner tubular member 36 through the distal guidewire port 47 and advanced proximally therein until it exits the proximal guidewire port 42. The proximal end of the guidewire 46 is held in place and the catheter is advanced over the guidewire through the patient's vascular system until the dilatation balloon 38 on the catheter is positioned across the stenotic region so that the stenosis can be dilated upon the inflation of the balloon. After the dilatation of the lesion, the balloon 38 is deflated and the catheter may be removed from the patient's artery. If other treatments are necessary, the catheter is slidably removed over the guidewire 46, leaving the guidewire in place so that other catheters can be advanced over the in place guidewire in a similar manner without the need for exchange wires or guidewire extensions.

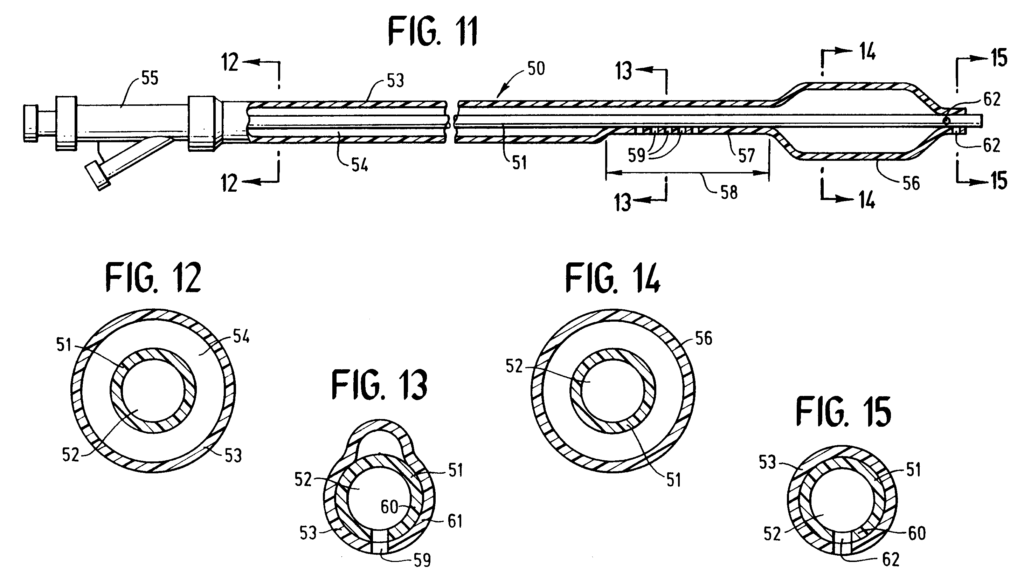

Figs. 11 through 15 illustrate yet another dilatation catheter not in accordance with the invention which provides for the perfusion of blood distal to the catheter during the dilatation of a stenotic lesion. The catheter includes the catheter shaft 50, an inner tubular member 51, with an inner lumen 52, an outer tubular member 53 which is disposed about the inner tubular member and which defines an annular lumen 54 located between the inner and outer tubular member in the proximal portion of the catheter shaft, an adapter 55 secured to the proximal ends of the inner and outer members, and a relatively inelastic balloon 56 which is secured by its distal end to the distal end of the inner tubular member 51. The outer tubular member 53 has a distal section 57, a length of 58 of which is secured to the exterior of the inner tubular member 51, as in the two catheters previously described. The above-described portion of this catheter has essentially the same structure as the catheters shown in Figs. 1-10. The distal section may be formed from the proximal skirt of the balloon 56 or may be formed from a separate tubular element with the proximal end of the balloon being secured to the distal end of the separate tubular element.

The dilatation catheter shown in Figs. 11-15 differs from the other catheters in that it has a plurality of perfusion ports 59 proximal to the balloon 56 which pass through the bonded walls 60 and 61 of the inner and outer tubular members 51 and 53 respectively and which are in fluid communication with the inner lumen 52 of the inner tubular member 51. Additionally, one or more perfusion ports 62 are provided distal to the balloon 56 through the wall 60 of the inner tubular member 51 and are in fluid communication with the inner lumen 52 extending therein. With this construction, when the balloon 56 is inflated *e.g*. during an angioplasty procedure, within a patient's vasculature, oxygenated blood is forced to pass through the proximal perfusion ports 59, through the inner lumen 52 and then out the distal perfusion ports 62 to provide oxygenated blood distal to the catheter and thereby avoid the generation of ischemic conditions in tissue downstream thereof or the aggravation of existing ischemic conditions. The transverse dimensions of the inner tubular member 51 within the secured section are preferably larger than in the catheters previously discussed to allow for an increased flow of blood therethrough.

The use of the catheter shown in Figs. 11-15 is essentially the same as the catheter shown in Figs. 1-5. The only essential difference is that the balloon 56 can be inflated for significantly longer periods *e.g.* typically about 20-30 minutes but possibly up to 5 hours or more, than the first described catheter because oxygenated blood is flowing to the issue distal to the inflated balloon.

The dilation catheter shown in Figs. 11-15 may be modified by providing a guidewire port at the proximal end of the section 58, proximal to the portion of the small diameter distal section 57 in which the proximal perfusion ports 59 are located, as shown in Figs 6-10. However, the guidewire port should preferably be spaced sufficiently far proximally from the portion of the bonded distal section 57 having the perfusion ports 59 so that the guidewire can be pulled proximally and remain within the inner lumen 52 while the balloon 56 is inflated during the dilatation but not interfere with the flow of blood through the perfusion ports 59 and 62 and the inner lumen 52. After the angioplasty procedure is completed, the guidewire can then be advanced distally through the inner lumen 52 and out the distal end thereof in order to maintain access to the lesion in case further treatment or diagnosis is necessary or desirable.

The use of the catheter with both perfusion ports and a proximal guidewire port as described above is essentially the same as the use of the dilatation catheter illustrated in Figs. 6-10, but with the additional advantage that long term dilatations are possible.

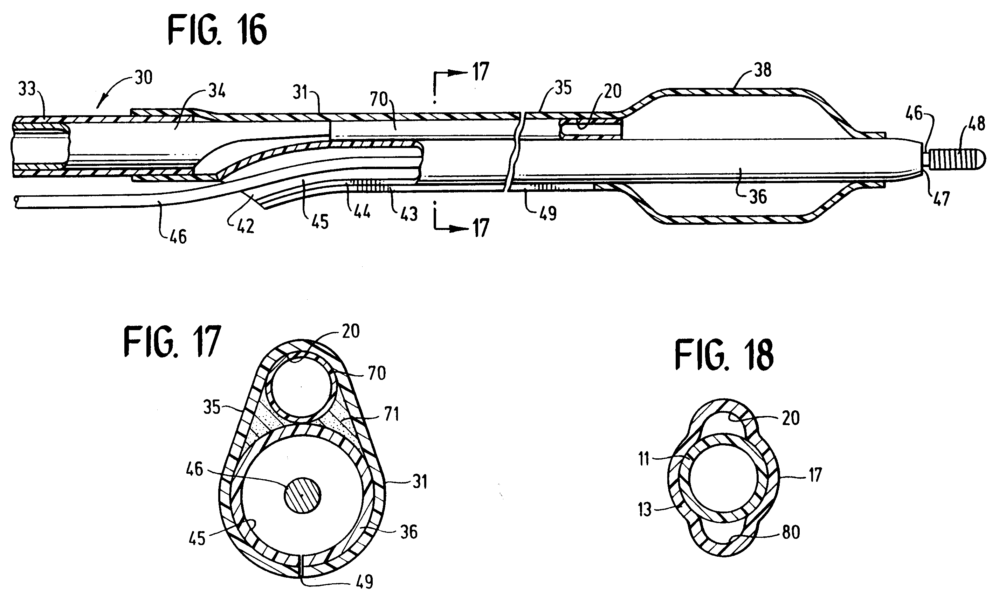

With those catheters which have a slit 49 in the secured section, such as shown in Figs 6-10, to facilitate the removal of the catheter from a guidewire, there is a tendency for the slit to open up when the pressure within the inflation lumen is raised to high levels to inflate the balloon 36 for purposes of dilatation. In these instances, the guidewire can extend through the expanded or opened slit 49 and be caught in the slit when it closes upon the deflation of the balloon so as to preclude independent movement of the guidewire and catheter. In the embodiment of the present invention illustrated in Figs. 16 and 17 the provision of the second inner tubular member (support tube) 70 within the inner lumen avoids this problem. The second inner tubular member, which forms the inflation lumen 40, prevents the expansion of the unsecured distal section 35 of the outer tubular member 31. Thus the expansion of the slit 49, upon the introduction of high pressure inflation fluid into the inflation lumen, is prevented. A filler 71 may be provided to eliminate any voids between the second inner tubular member 70 and the unsecured portion of the distal tubular section 35. Preferably, the second inner tubular meter 70 is formed of polyimide but tubes formed of other polymers and metals such as superelastic NiTi alloys may be used. The rest of the catheter shown in Figs. 16 and 17 is essentially the same as shown in Figs. 6-10 and the corresponding parts are numbered the same.

The above-described catheters may be made by conventional techniques well known to those skilled in the art. Many suitable techniques are described in the references identified herein. The distal section may be formed by heat shrinking the portion of the outer tubular member which forms the distal section onto the underlying inner member. A mandrel (not shown) is disposed in the space between the inner and outer tubular member, so that, upon the heat shrinking of the outer tubular member, an inflation lumen is formed through the distal section which is in fluid communication with the lumen in the proximal portion of the catheter shaft and the interior of the balloon. The heat shrinking secures the distal section of the outer tubular member to the inner tubular member. Other means of bonding such as heat bonding or adhesives may be used. A mandrel may also be inserted into the inner lumen of the inner tubular member to support the latter during the heat shrinking of the outer tubular member thereon. Alternate methods may be employed to make the distal section. For example, the distal section of the outer tubular member may be preformed and then be adhesively bonded to the exterior of the inner tubular member 36.

As shown in Fig. 18, other lumens 80, similar to the inflation lumen may be formed in the catheter shaft, by employing multiple mandrels when heat shrinking the outer tubular member onto the exterior of the inner tubular member. In such catheters it is preferred to have the secured section extend along the entire length of the catheter so that the adapter on the proximal end would be connected to the all of the individual lumens. In this manner the extra lumens may be employed to deliver drugs or other therapeutic fluids or be used as an additional inflation lumen.

The various components of the catheters and guidewires of the invention can be formed from a wide variety of conventional materials. The inner and outer plastic tubular members may be made from polyethylene, polyimide, polyvinyl chloride and other suitable plastic materials. The hypotubing may be formed of stainless steel, NiTi superelastic alloys or other suitable materials. Composite materials may also be used. The balloon may be made from polyethylene, polyethylene terephthalate, olefinic ionomers such as Surlyn® sold by E.I. DuPont, deNemours & Co. and other polymers and other materials.

The dimensions of the catheters generally follow the dimensions of conventional intravascular catheters. For coronary use the length is typically about 135 cm and the maximum outer diameter of the outer tubular member is about 0.02 to about 0.06 inch (0.51-1.52 mm). In a presently preferred embodiment, the distal secured section of the outer tubular member is long enough (*e.g.* preferably about 10 to about 40 cm) to ensure that it is the only portion of the catheter body proximal to the balloon which exits the guiding catheter and enters the patient's coronary anatomy during intravascular procedures. In other embodiments, the secured section may extend along essentially the entire length of the catheter shaft. The transverse dimensions of the catheter may be larger with catheters for use in peripheral arteries and other locations.

## Claims

1. An elongated catheter for performing an angioplasty procedure comprising:
a) an elongated catheter shaft (30) which has a distal shaft section with
a first inner tubular member (36) having a distal end, a port (47) in the distal end and a guidewire-receiving inner lumen (45) in fluid communication with the port (47) in the distal end, and
an outer tubular member (31) being disposed about the first inner tubular member and having a portion of its inner periphery, along a length (39) of the distal shaft section, secured to the exterior of the first inner tubular member; and
b) a dilatation balloon (38) on the distal shaft section distal to at least a portion of said length and having an interior chamber therein;
characterized in that
the catheter further comprises a second inner tubular member (70) within the distal shaft section which has a distal end and a first port in the distal end, which is parallel to and off-set from the first inner tubular member, which extends to a location in the distal shaft section spaced proximally from the distal end of the first inner tubular member, which has an inflation lumen (40) extending therein in fluid communication with the interior chamber of the dilatation balloon through the port in the distal end of the second inner tubular member and which has a portion of the inner periphery of the outer tubular member along said length secured to an exterior portion of the second inner tubular member;
the catheter shaft (30) is provided with a second port which is located closer to the dilation balloon (38) than the proximal end of the catheter shaft and which is in communication with the inner lumen (45) of the first inner tubular member (36);
the distal shaft section has along the length thereof one transverse dimension in a first direction substantially larger than a second transverse dimension in a second direction perpendicular to the first direction; and
from 5% to 90% of the inner periphery of the outer tubular member is secured to the exterior of the first inner tubular member.

2. A catheter according to claim 1, wherein the dilation balloon (38) has a distal end secured to the distal end of the first inner tubular member (36).

3. A catheter according to claim 1 or claim 2, wherein a portion of the catheter body proximal to the secured portion is stiffer than the distal section.

4. A catheter according to claim 3, wherein a portion of the outer tubular member (31) proximal to the secured portion thereof is formed of hypotubing to increase the stiffness thereof.

5. A catheter according to any one of the preceding claims, including a peel-away slit extending distally from the second port and terminating proximal to the dilation balloon (38).

6. A catheter according to any one of the preceding claims, wherein the outer tubular member (31) is provided with a plurality of unsecured portions along said length and wherein an inner lumen extends through each of said plurality of unsecured portions.

7. A catheter according to claim 9, wherein the dimension in the first transverse direction is at least 15% greater than the dimension in the second transverse direction.

8. A catheter according to claim 9, wherein the dimension in the first transverse direction is at least 25% greater than the dimension in the second transverse direction.

9. A catheter according to any one of the preceding claims, wherein the first and second lumens (40,45) in the distal shaft section are in a stacked configuration.

## Patentansprüche

1. Langgestreckter Katheter zum Durchführen eines Angioplastiebehandlungsverfahrens, mit:
a) einem länglichen Katheterschaft (30), der einen distalen Schaftabschnitt aufweist, mit
einem ersten inneren rohrförmigen Teil (36), das ein distales Ende, eine Öffnung (47) im distalen Ende und einen inneren Hohlraum (45) zur Aufnahme eines Führungsdrahtes aufweist, der in Strömungsmittelkommunikation mit der Öffnung (47) im distalen Ende steht, und
einem äußeren rohrförmigen Teil (31), das um das erste innere rohrförmige Teil angeordnet ist und einen Abschnitt seines Innenumfangs längs eines Längenabschnitts (39) des distalen Schaftabschnitts aufweist, der an der Außenseite des ersten inneren rohrförmigen Teils befestigt ist; und
b) einem Aufweitungsballon (38) am distalen Schaftabschnitt, der in Bezug auf wenigstens einen Teil des Längenabschnitts distal angeordnet ist und im Inneren eine Innenkammer aufweist;
dadurch gekennzeichnet,
daß der Katheter ferner ein zweites inneres rohrförmiges Teil (70) innerhalb des distalen Schaftabschnitts aufweist, das ein distales Ende und eine erste Öffnung im distalen Ende aufweist, das parallel zum ersten inneren rohrförmigen Teil und gegenüber diesem versetzt ist, sich zu einer Stelle im distalen Schaftabschnitt erstreckt, der proximal vom distalen Ende des ersten inneren rohrförmigen Teils beabstandet ist, einen Aufpumphohlraum (40) aufweist, der sich darin in Strömungsmittelkommunikation mit der inneren Kammer des Aufweitungsballons durch die Öffnung im distalen Ende des zweiten inneren rohrförmigen Teils hindurch erstreckt und einen Abschnitt des Innenumfangs des äußeren rohrförmigen Teils längs des Längenabschnitts aufweist, der an einem äußeren Abschnitt des zweiten inneren rohrförmigen Teils befestigt ist;
der Katheterschaft (30) mit einer zweiten Öffnung versehen ist, die näher am Aufweitungsballon (38) gelegen ist als das proximale Ende des Katheterschafts und mit dem inneren Hohlraum (45) des ersten rohrförmigen Teils (36) kommuniziert;
der distale Schaftabschnitt längs seines Längenabschnitts eine Querabmessung in einer ersten Richtung aufweist, die wesentlich größer ist als eine zweite Querabmessung in einer zweiten Richtung senkrecht zur ersten Richtung; und
von 5% bis 90% des Innenumfangs des äußeren rohrförmigen Teils an der Außenseite des ersten inneren rohrförmigen Teils befestigt ist.

2. Katheter nach Anspruch 1, bei welchem der Aufweitungsballon (38) ein distales Ende hat, das am distalen Ende des ersten inneren rohrförmigen Teils (36) befestigt ist.

3. Katheter nach Anspruch 1 oder Anspruch 2, bei welchem der Abschnitt des Katheterkörpers, der zum befestigten Abschnitt proximal liegt, steifer ist als der distale Abschnitt.

4. Katheter nach Anspruch 3, bei welchem ein Abschnitt des äußeren rohrförmigen Teils (31), der proximal zu dem befestigten Abschnitt liegt, aus Unterrohr gebildet ist, um seine Steifigkeit zu erhöhen.

5. Katheder nach einem der vorangehenden Ansprüche, mit einem Abschälschlitz, der sich distal von der zweiten Öffnung erstreckt und proximal zum Aufweitungsballon (38) endet.

6. Katheter nach einem der vorangehenden Ansprüche, bei welchem das äußere rohrförmige Teil (31) mit einer Vielzahl nichtbefestigter Abschnitte längs des Längenabschnitts versehen ist, und bei welchem sich ein innerer Hohlraum durch jeden aus der Vielzahl von nichtbefestigten Abschnitten erstreckt.

7. Katheter nach Anspruch 9, bei welchem die Abmessung in der ersten Querrichtung mindestens um 15% größer als die Abmessung in der zweiten Querrichtung ist.

8. Katheter nach Anspruch 9, bei welchem die Abmessung in der ersten Querrichtung mindestens um 25% größer als die Abmessung in der zweiten Querrichtung ist.

9. Katheter nach einem der vorangehenden Ansprüche, bei welchem der erste und zweite Hohlraum (40,45) im distalen Schaftabschnitt in geschichteter Anordnung vorliegen.

## Revendications

1. Cathéter allongé pour l'exécution d'opérations d'angioplastie angiologique, comprenant :
a) un corps allongé de cathéter (30) qui présente une section distale de corps, incluant
un premier élément tubulaire intérieur (36) ayant une extrémité distale, un orifice (47) à l'extrémité distale et un passage intérieur (45) de logement d'un fil métallique de guidage, qui est en communication fluidique avec l'orifice (47) de l'extrémité distale et
un élément tubulaire extérieur (31) qui est disposé autour du premier élément tubulaire intérieur et qui comporte une partie de sa périphérie intérieure située sur une longueur (39) de la section distale de corps qui est fixée sur l'extérieur du premier élément tubulaire intérieur ; et
b) un ballonnet de dilatation (38), situé sur la section distale du corps, qui est sur le côté distal par rapport à au moins une partie de ladite longueur et qui présente à l'intérieur une chambre interne ;
caractérisé en ce que
le cathéter comprend par ailleurs un deuxième élément tubulaire intérieur (70) qui est situé dans la section distale du corps, qui comporte une extrémité distale et un premier orifice à l'extrémité distale, qui est parallèle à et décalé par rapport au premier élément tubulaire intérieur, qui se prolonge jusqu'à un emplacement situé dans la section distale du corps et distant sur le côté proximal de l'extrémité distale du premier élément tubulaire intérieur, qui comporte un passage de gonflement (40) situé à l'intérieur et en communication fluidique avec la chambre interne du ballonnet de dilatation par l'orifice de l'extrémité distale du deuxième élément tubulaire intérieur et qui présente une partie de la périphérie intérieure de l'élément tubulaire extérieur, située sur ladite longueur, qui est fixée à une partie extérieure du deuxième élément tubulaire intérieur ;
le corps (30) du cathéter comporte un deuxième orifice qui est placé plus près du ballonnet de dilatation (38) que l'extrémité proximale du corps du cathéter et qui est en communication avec le passage interne (45) du premier élément tubulaire intérieur (36) ;
la section distale du corps présente sur sa longueur une dimension transversale dans une première direction qui est sensiblement plus grande qu'une deuxième dimension transversale dans une deuxième direction perpendiculaire à la première direction ; et
de 5% à 90% de la périphérie interne de l'élément tubulaire extérieur sont fixés sur l'extérieur du premier élément tubulaire interne.

2. Cathéter selon la revendication 1, dans lequel le ballonnet de dilatation (38) a une extrémité distale qui est fixée à l'extrémité distale du premier élément tubulaire intérieur (36).

3. Cathéter selon la revendication 1 ou la revendication 2, dans lequel une partie du corps du cathéter située de manière proximale par rapport à la partie fixée est plus rigide que la section distale.

4. Cathéter selon la revendication 3, dans lequel une partie de l'élément tubulaire extérieur (31) qui est située de manière proximale par rapport à sa partie fixée est formée avec mise en place d'un sous-tubage pour en accroître la rigidité.

5. Cathéter selon l'une quelconque des revendications précédentes, comprenant une fente pouvant être dépouillée, située sur le côté distal par rapport au deuxième orifice et qui aboutit sur le côté proximal du ballonnet de dilatation (38).

6. Cathéter selon l'une quelconque des revendications précédentes, dans lequel l'élément tubulaire extérieur (31) comporte plusieurs sections non fixées sur ladite longueur et dans lequel un passage interne est réalisé dans chacune desdites plusieurs parties non fixées.

7. Cathéter selon la revendication 9, dans lequel la dimension dans la première direction transversale est d'au moins 15% supérieure à la dimension dans la deuxième direction transversale.

8. Cathéter selon la revendication 9, dans lequel la dimension dans la première direction transversale est d'au moins 25% supérieure à la dimension dans la deuxième direction transversale.

9. Cathéter selon l'une quelconque des revendications précédentes, dans lequel les premier et deuxième passages (40, 45) situés dans la section distale du corps sont à une conformation de superposition.
